# EUROPÄISCHE PATENTANMELDUNG

(11) **EP 1 254 648 A1**
(43) Veröffentlichungstag der Anmeldung: **06.11.2002**
(21) Anmeldenummer: 01250157.3
(22) Anmeldetag: 05.05.2001
(51) Int. Cl.: A61F 5/56

(54) **Vorrichtung zum Verhindern des Schnarchens**

(71) Anmelder: Golriz Fard, Bijan, 30519 Hannover (DE)
(72) Erfinder: Golriz Fard, Bijan, 30519 Hannover (DE)
(74) Vertreter: Seewald, Jürgen, Dipl.-Ing.

(57) **Zusammenfassung**

Die vorliegende Erfindung betrifft eine Vorrichtung zum Verhindern des Schnarchens mit einem Kopfkissen, einem Schallsensor zum Aufnehmen der Schnarchgeräusche und einer durch die Schnarchgeräusche aktivierbaren Kontrolleinheit, die eine Lageveränderung des Kopfes eines Schläfers durch Bewegung des Kopfkissens steuert. Aufgabe der Erfindung ist es, eine Vorrichtung dieser Art zur Verfügung zu stellen, die einfach aufgebaut ist, den Schlafkomfort nicht oder nur unwesentlich beeinträchtigt und dennoch ein Schnarchen wirksam verhindert. Gelöst wird diese Aufgabe dadurch, daß das Kopfkissen (5) ein in Kammern (1 - 4) unterteiltes Luftkissen ist und daß der Luftdruck der Kammern (1 - 4) durch die Kontrolleinheit in Verbindung mit mindestens einer Luftdruckquelle und einer Luftdruck-Reduziereinheit steuerbar ist.

## Beschreibung

Die vorliegende Erfindung betrifft eine Vorrichtung zum Verhindern des Schnarchens gemäß dem Anspruch 1.

In der DE 37 27 258 C2 ist eine Vorrichtung zum Verhindern des Schnarchens offenbart. Sie besteht aus einem Gehäuse, welches als Unterlage unter ein Kopfkissen legbar ist. Das Gehäuse besitzt einen drehbar mit seinen Seitenwänden verbundenen Dekkel. Im Gehäuse ist ein Exzenterantrieb für den Deckel angeordnet, der seinerseits durch einen Elektromotor angetrieben wird. Weiterhin ist ein elektronisches System vorgesehen, das die über ein Mikrophon aufgenommenen Schnarchgeräusche in elektrische Energie für den Elektromotor umwandelt. Alle genannten Komponenten sind innerhalb des Gehäuses angeordnet. Der geschilderte Antrieb des Gehäusedeckels versetzt diesen in eine rollende bzw. taumelnde Bewegung, die solange fortgesetzt wird, bis das Schnarchen aufhört.

Nachteilig an dieser Lösung ist, daß das notwendigerweise harte Gehäuse mit seinem ebenfalls harten Deckel den Schlafkomfort wesentlich einschränkt.

In DE 41 37 631 A1 ist eine Einrichtung zum randseitigen Anheben einer Matratze beschrieben. Diese Einrichtung besteht aus einem mindestens auf einem der beiden Längs-Randbereiche der Matratze angeordneten, mit Luft aufblähbaren Hohlkörper. In der Grundstellung ist dieser mindestens eine Hohlkörper leer, d.h. er liegt flach unter der Matratze. Bei Bedarf, d.h. wenn über ein Mikrophon ein Schnarchgeräusch aufgenommen wird, wird ein Pumpenaggregat eingeschaltet, welches den Hohlkörper schnell füllt, da dieser ein relativ geringes Volumen besitzt. Durch das Anheben des wenigstens einen Randbereichs der Matratze wird die Lage des Schlafenden geändert, so daß das Schnarchen aufhört. Ist das nicht der Fall, muß dieser Vorgang wiederholt werden. Nachteilig an dieser Lösung ist, daß durch die Bewegung des gesamten Körpers der Schlaf nachhaltig gestört wird.

Aus dem DE 91 00 663 U1 ist eine Vorrichtung zum Reduzieren des Schnarchens bekannt, die ein Abrollgebilde aufweist, das sich im Kissenbereich in Verlängerung der Längs-Mittellinie des Körpers eines Schläfers erstreckt. In einer Ausführungsform besteht dieses Abrollgebilde aus einem aufblasbaren Schlauch. Beginnt ein Schläfer zu schnarchen, so wird das über einen Geräuschsensor erfaßt, der eine Pumpe einschaltet, die den Schlauch aufpumpt. Da der sich wölbende Schlauch gegen den Hinterkopf des Schläfers drängt, wendet dieser seinen Kopf zur Seite. An Stelle des Geräuschsensors kann auch ein Drucksensor treten, der in dem Kopfkissen angeordnet ist. Es bleibt dabei unklar, wie dieser Drucksensor wirken soll. Entweder wirkt er ständig, weil der Kopf des Schläfers ihn ständig berührt, oder er wirkt nur dann, wenn der Kopf des Schläfers in eine andere Position bewegt und der Sensor dabei berührt wird. Beides ist unsinnig, da der Schläfer im ersteren Fall nicht zur Ruhe kommen würde, und der Sensor im zweiten Fall erst dann auslösen würde, wenn das, was er bewirken soll, nämlich eine Kopfbewegung, bereits vollzogen ist.

Die DE 195 35 232 C2 betrifft eine verstellbare Stütze für Kopfkissen mit einer im wesentlichen rechteckigen, beweglichen Auflagefläche für das Kopfkissen. In einer Ausführungsform dieser Stütze sind unter allen vier Seiten der Auflagefläche Huborgane aus pneumatischen Druckkissen angeordnet, die über von einer Steuereinrichtung betätigbare Ventile mit einer gemeinsamen Pumpe verbunden sind. Durch Betätigen der Pumpe wird eines der Huborgane mit Luft gefüllt und auf diese Weise die entsprechende Seite der Auflagefläche angehoben. Dadurch wird eine Verlagerung des Körpers in eine Seitenlage angeregt bzw. der Kopf hinten oder vorn angehoben. Schnarcher werden auf diese Weise, gegebenenfalls nach mehreren Positionswechseln, in eine Schlafstellung gebracht, in der das Schnarchen aufhört. Die Pumpe kann über einen akkustischen Sensor eingeschaltet werden.

Schließlich ist auch aus der JP 10-85108 A eine Antischnarchvorrichtung in Form eines Kopfkissens bekannt, welches aus mehreren, nebeneinander angeordneten Luftkammern besteht. Diese Luftkammern werden unterschiedlich aufgeblasen und wieder entlüftet, wodurch eine Bewegung des Kopfes eines Schlafenden erreicht wird.

Allen bekannten, mit Luftkammern arbeitenden Antischnarchvorrichtungen ist gemeinsam, daß die Beaufschlagung der Luftkammern willkürlich erfolgt, d.h. daß auch Luftkammern beaufschlagt werden, die keine Wirkung erzielen. Aufgabe der vorliegenden Erfindung ist es, hier Abhilfe zu schaffen.

Gelöst wird diese Aufgabe bei einer gattungsgemäßen Vorrichtung zum Verhindern des Schnarchens dadurch, daß die Kontrolleinheit die Lage des Kopfes eines Schläfers über die aus der Gewichtskraft des Kopfes resultierende Druckerhöhung in den vom Kopf belegten Kammern erfaßt.

Durch diese Lösung wird das vorhandene Kammersystem des Kopfkissens dazu benutzt, die örtliche Lage des Kopfes einer schlafenden Person zu erfassen. Dabei wird die Tatsache ausgenutzt, daß durch die Gewichtskraft des Kopfes Druck auf die jeweilig beteiligten Kammern ausgeübt wird. Die daraus resultierende Druckerhöhung in einer Kammer bzw. mehreren Kammern wird durch die Kontrolleinheit erfaßt. Diese Sensorik gestattet es, über die Kontrolleinheit gezielt die Kammern anzusteuern, deren Druckluft-Beaufschlagung bzw. -Reduzierung den gewünschten Effekt (Kopfbewegung) bringt. Eine Beaufschlagung von wegen ihrer Lage zum Kopf unwirksamen Kammern wird damit vermieden. Diese Sensorik kann auch dazu benutzt werden, statistisch die örtliche Lage des Kopfes zu erfassen, in der das Schnarchen besonders häufig ausgelöst wird. Weiterbildungen der vorliegenden Erfindung ergeben sich aus den übrigen Unteransprüchen.

Die Erfindung wird nachstehend anhand eines Ausführungsbeispiels näher erläutert. In der dazugehörigen, rein schematischen Zeichnung zeigt:
- Fig. 1: eine Seitenansicht eines erfindungsgemäßen Kopfkissens in normaler Schlafstellung,
- Fig. 2: eine Vorderansicht dieses Kopfkissens in normaler Schlafstellung,
- Fig. 3: eine Draufsicht auf das Kopfkissen,
- Fig. 4: eine Darstellung gemäß Fig. 1 bei aktiviertem Kopfkissen mit nach rechts fallender Kopfkissen-Oberfläche,
- Fig. 5: eine Darstellung gemäß Fig. 2 bei aktiviertem Kopfkissen und nach links fallender Kopfkissen-Oberfläche,
- Fig. 6: eine Darstellung gemäß Fig. 1 bei aktiviertem Kopfkissen mit von hinten nach vorn fallender Kopfkissen-Oberfläche,
- Fig. 7: eine Darstellung gemäß Fig. 1 bei aktiviertem Kopfkissen mit von vorn nach hinten fallender Kopfkissen-Oberfläche, und
- Fig. 8: einen perspektivischen Blick von schräg vorn und oben auf das Kopfkissen in normaler Schlafstellung.

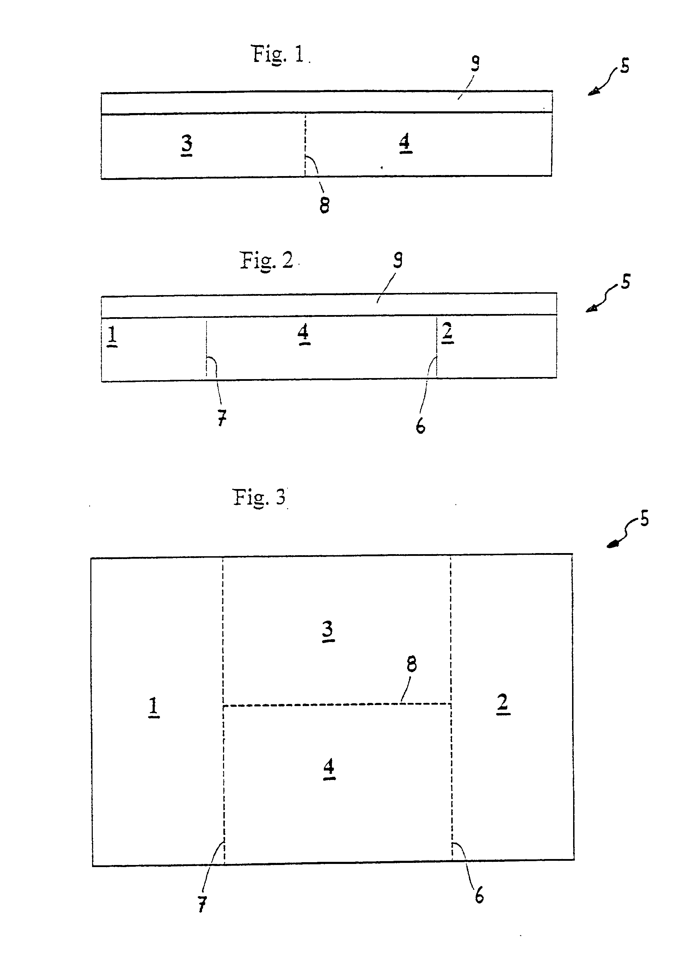

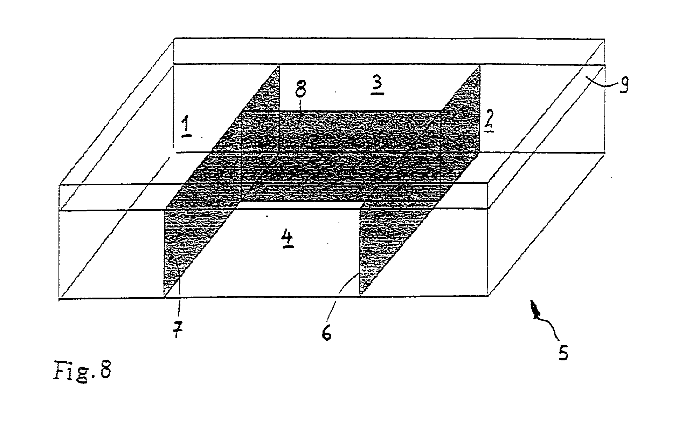

Das Kopfkissen 5 dieses Ausführungsbeispiels besitzt vier Luftkammern 1 bis 4, wie aus den Darstellungen gemäß den Fig. 3 und 8 hervorgeht. Es sind zwei seitliche Kammern 1, 2 vorgesehen, die sich jeweils über die gesamte Längsseite des Kopfkissens 5 erstrecken. Zwischen diesen Kammern 1, 2 sind zwei dazu quer ausgerichtete Kammern 3, 4 vorgesehen, die die Rest-Grundfläche des Kopfkissens 5 einnehmen. Die Kammern 1 bis 4 sind durch flexible Trennwände 6, 7 und 8 voneinander luftdicht getrennt.

Oberhalb der Kammern 1 bis 4 ist eine Deckschicht 9 vorgesehen, die eine weiche Konsistenz besitzt und zum Beispiel aus Schaumstoff bestehen kann. Vorteilhaft ist es aber, wenn diese Schicht 9 aus einem mit einem Gel gefüllten Raum besteht, da eine derartige Schicht 9 der Fixierung des Kopfes dient sowie ein sehr angenehmes Auflagegefühl vermittelt. Um eine gute Belüftung des Kopfbereiches zu gewährleisten, kann die Oberfläche der Schicht 9 perforiert sein. Auf diese Oberfläche kann auch noch zusätzlich eine dünne Schicht aus Schaumstoff aufkaschiert sein.

Jede der Kammern 1 bis 4 besitzt einen Anschluß, der mit einer von einem Luftdruckkompressor kommenden Leitung verbunden ist, sowie ein Entlüftungsventil. Diese Vorrichtungskomponenten sind nicht dargestellt. Ebenfalls nicht gezeigt sind eine Kontrolleinheit sowie ein mit dieser verbundenes Mikrophon. Dieses Mikrophon ist in der Nähe des Schlafenden angeordnet. Bei auftretenden Schnarchgeräuschen aktivieren die entsprechenden Signale des Mikrophons die Kontrolleinheit, die wiederum das Pumpaggregat sowie gegebenenfalls die Entlüftungsventile ansteuert, d.h. an- oder ausschaltet bzw. öffnet oder schließt.

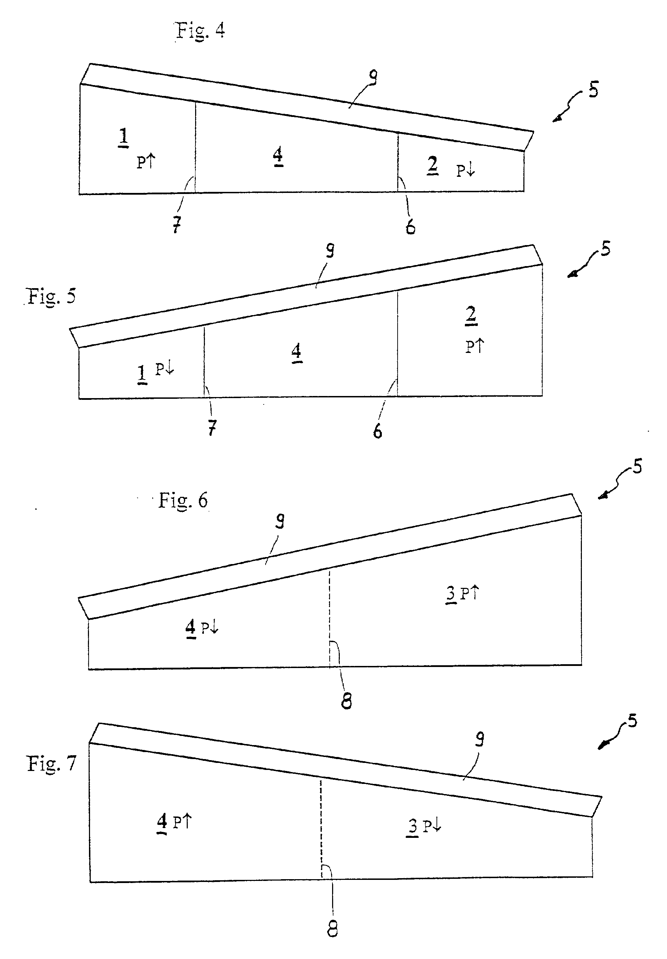

Die in diesem Ausführungsbeispiel gewählte Unterteilung des Kopfkissens 5 gewährleistet eine Bewegung des Kopfes eines Schlafenden in allen Richtungen. Wird der Druck in Kammer 1 erhöht und in Kammer 2 verringert (Fig. 4), so bewirkt das eine Drehung des Kopfes nach links. Bei umgekehrter Beaufschlagung der Kammern 1 und 2 (Fig. 5) wird ein nach links weisender Kopf nach rechts gedreht (von der Position eines Schlafenden aus betrachtet).

Eine Erhöhung des Drucks in Kammer 4 und eine Druckreduzierung in Kammer 3 verursacht eine Retroversion (Rückwärtsbewegung = Streckung des Kopfes) (Fig. 3). Bei einer umgekehrten Beaufschlagung der Kammern 4 und 3 wird der Kopf nach vorn gebeugt (Fig. 6) (Anteversion).

Die oben beschriebenen Bewegungen können natürlich auch kombiniert werden. Je mehr Kammern vorhanden sind, umso größer sind die Möglichkeiten der Formänderung der Kopfkissen-Oberfläche.

## Patentansprüche

1. Vorrichtung zum Verhindern des Schnarchens mit einem Kopfkissen, einem Schallsensor zum Aufnehmen der Schnarchgeräusche und einer durch die Schnarchgeräusche aktivierbaren Kontrolleinheit, die eine Lageveränderung des Kopfes eines Schläfers durch Bewegung des Kopfkissens steuert, wobei das Kopfkissen ein in Kammern unterteiltes Luftkissen ist, und - der Luftdruck der Kammern durch die Kontrolleinheit in Verbindung mit mindestens einer Luftdruckquelle und einer Luftdruck-Reduziereinheit steuerbar ist, **gekennzeichnet dadurch, daß** die Kontrolleinheit die Lage des Kopfes eines Schläfers über die aus der Gewichtskraft des Kopfes resultierende Druckerhöhung in den vom Kopf belegten Kammern erfaßt.

2. Vorrichtung nach Anspruch 1, **dadurch gekennzeichnet, daß** zwei seitliche, sich über das gesamte Kopfkissen (5) erstreckende und zwei den Bereich zwischen diesen seitlichen Kammern (1, 2) ausfüllende und quer zu diesen seitlichen Kammern (1, 2) angeordnete Kammern (3, 4) vorgesehen sind.

3. Vorrichtung nach Anspruch 1 oder Anspruch 2, **dadurch gekennzeichnet, daß** die Kammern (1 - 4) nach oben durch eine Gelschicht (9) abgedeckt sind.
